# EUROPEAN PATENT APPLICATION

(11) **EP 1 284 149 A2**
(43) Date of publication of application: **19.02.2003**
(21) Application number: 02255461.2
(22) Date of filing: 05.08.2002
(51) Int. Cl.: A61N 1/372, A61N 1/37

(54) **Ambulatory recording device for use with an implantable cardiac stimulating device**

(30) Priority: 07.08.2001 US 924897
(71) Applicant: Pacesetter, Inc., Sylmar, CA 91342 (US)
(72) Inventor: Poore, John W., South Pasadena, CA 91030 (US)
(74) Representative: Roberts, Gwilym Vaughan

(57) **Abstract**

An ambulatory recording device (100) that is worn by the patient for extended period of time and communicates with an implanted cardiac stimulation device (210). The ambulatory recording device (100) communicates via the telemetry circuit (156) of the implanted cardiac stimulation device (210) and receives data from the implanted cardiac stimulation device (210) indicative of the performance of the implanted cardiac stimulation device (210) and of electrical activity of the heart (102). This data can be downloaded from the ambulatory recording device (100) to a local or remote location to permit subsequent evaluation of the data to assess the performance of the implantable cardiac stimulation device (210).

## Description

The present invention relates to implantable cardiac devices, such as implantable pacemakers and implantable cardioverter defibrillators (ICDs), and, more particularly, concerns a system for recording data from a cardiac stimulation device implanted in a patient, such as intracardiac electrogram (IEGM) signals, marker data, sensor data, and other stored signals and data, over an extended period of time to permit subsequent evaluation of the operation of the implanted device.

Implantable cardiac stimulation devices, such as pacemakers, ICD's, or devices that incorporate the functionality of both pacemakers and ICD's have become increasingly more complex. The increase in complexity is the result of improved components and processes that enable more sophisticated therapies to be provided to the heart of the patient. As a result of these more sophisticated therapies, the algorithms that are used by the implantable cardiac stimulation devices in order to determine when and how to deliver therapeutic electrical stimulation to the heart have also become more complex.

In particular, given the increased sensing and processing capabilities of pacemakers and ICD's, the delivery of therapeutic electrical stimulation, such as pacing pulses or therapeutic electrical waveforms to terminate various arrhythmias, can be significantly varied, such that the therapy is more tailored for the specific sensed conditions of the heart at the time of the delivery of the therapeutic electrical stimulation. One consequence of the more sophisticated algorithms for delivering therapeutic electrical stimulation to the heart of a patient is that it is more difficult to ensure that the implantable cardiac stimulation device therapy is beneficial to a particular patient. For example, it is essential to evaluate the efficacy of an atrial fibrillation or anti-tachycardia pacing therapy.

Unfortunately, it is often very difficult for the implanting or treating physician to determine whether the algorithms are responding properly or advantageously and sensors over extended periods of time since an arrhythmia may not occur while the patient is in the physician's office. An implantable cardiac stimulation device attempts to correct abnormal heart function, which may only occur at sporadic intervals. Without having data indicative of how the device performs when the actual heart abnormality occurs, it is often very difficult for the treating or implanting physician to determine whether the device settings are appropriately set or if the algorithm is appropriate for the patient.

Hence, there is a need for some type of monitoring device or system that is capable of monitoring the performance of a cardiac stimulation device that has been implanted in the patient. To address this particular need, many implantable cardiac stimulation devices have been equipped with memory and are configured to record data when the device has detected an abnormality in heart function. However, the recording capability of the implantable cardiac stimulation device is generally very limited due to the limitations on size and power associated with the implanted devices. Hence, implanted cardiac stimulation devices, such as pacemakers and ICD's, are unable to record data over an extended period of time which is often necessary to be able to properly evaluate whether the implanted cardiac stimulation device is functioning appropriately or if therapy is beneficial.

There are some external systems, which are designed to monitor patients over extended periods of time. A typical example is a well-known Holter monitor, which is worn by the patient over an extended period of time, such as over a 24-hour period, or an event recorder that records episodes over an extended period of time. However, the Holter monitor generally only monitors the function of the patient's heart using skin electrodes and does not monitor the performance of the implanted cardiac stimulation device, the data that the device is receiving and the manner in which the device is evaluating the data. Hence, while a Holter monitor may provide an indication as to how the patient's heart is functioning, it does not provide much indication as to how the implanted cardiac stimulation device is interpreting or is viewing the heart as functioning. Hence, Holter monitors provide very limited information with respect to the actual operation of the implanted cardiac stimulation device and its interpretation and response to the functioning of the heart.

There have also been systems, which are designed to interrogate the implanted cardiac device via the telemetry circuit associated therewith so as to be able to record device data. One such system is disclosed in U. S. Patent No. 5,336,245 to Adams et al. While this patent discloses a system that is actually receiving data from the implanted device, this system is only used for a limited period of time, such as when the patient is actually at the doctor's office, and is incapable of recording data over an extended period of time. Consequently, this system is not likely to observe the implanted cardiac stimulation devices' responses to abnormal heart conditions unless such heart conditions are occurring while the patient is connected to the system.

Hence, it will be appreciated from the foregoing that there is a need for a system that is capable of recording not only heart function, but also implanted cardiac stimulation device information and its interaction with the heart over an extended period of time in order to allow implanting or treating medical professionals to evaluate the performance of the device. To this end, there is a need for a monitoring system that obtains signals indicative of not only the function of the patient's heart, but also signals indicative of how the implanted device is evaluating the signals that it is receiving about the heart function or other physiological effects within the patient's body.

The aforementioned needs are satisfied by the ambulatory recording device for monitoring the performance of an implantable cardiac stimulation device of the present invention. The device of the present invention is positioned within a housing worn external to the patient's body and includes a communication interface that communicates with and receives data from the implanted cardiac stimulation device. The device further includes a storage system that stores the received data and a processor coupled to the communications interface in the storage system that triggers the storage of the received data in the storage system for a predetermined period of time. In one aspect, the device is adapted to permit the stored data to be transferred to a remote system for subsequent analysis.

As the device is designed to be worn by the patient and as the device is external to the patient's body, larger capacity memories and batteries can be used than those available to implanted devices. Data can thus be recorded for an extended period of time, e.g., twenty-four continuous hours. Moreover, because the device is communicating with the cardiac stimulation device, the system can receive both the signals that are being received by the cardiac stimulation device, and also marker signals and state signals that are indicative of the functioning of the cardiac stimulation device in response to the signals being provided to the cardiac stimulation device. Hence, the data can thus be used to evaluate how the cardiac stimulation device is responding to the signals that it is receiving indicative of the patient's heart function or other physiological characteristics of the patient. In this way, the data can thus be used to modify the programming of the cardiac stimulation device so as to more appropriately tailor the delivery of pacing pulses or waveforms from the cardiac stimulation device to the heart.

In one embodiment, the recording device includes an interface that permits transferring of the data stored therein to an external system that archives and/or processes the stored data. In one embodiment, the memory system includes memory cartridges that can be removed from the memory system to allow transfer of data to another location, e.g., it can be mailed to a clinician for data transfer. In another application, the memory system is adapted to permit electronic downloading of the data to a remote location via a communications system such as via a network. In this way, data can be accumulated over an extended period of time and then periodically transferred to a remote location, such as a doctor's office, to permit evaluation of the data without requiring the patient to actually physically travel to the remote location.

In another embodiment, the recording device includes an external monitor, such as an ECG monitor or other external sensor that obtains an external signal, such as a skin ECG or other external signals, such as pressure relative to an internal pressure sensor in the implantable device. The external signal is simultaneously stored in the ambulatory recording device such that the system is receiving both a signal provided by the cardiac stimulation device and also corresponding external signals. In this way, the signal that the cardiac stimulation device is providing can be compared to the external signal for evaluation purposes.

In another aspect, the present invention comprises a method for monitoring the performance of an implantable cardiac stimulation device. In this aspect, the method comprises mounting a receiver external to the patient's body and delivering signals from the cardiac stimulation device to the receiver. The method further comprises recording the signals received by the receiver in a recording device, such that the signals from the cardiac stimulation device can be recorded for an extended period of time.

In one particular embodiment, the method further comprises transferring the recorded signals to an external location. In one particular embodiment, the transferring of the information to an external device comprises removing a removable memory cartridge, such as a flash memory card, from a housing containing the system and forwarding the removable memory cartridge to the external location. In another embodiment, transferring the stored data comprises periodically downloading the stored data via a communications interface, such as by direct connection to a PC or via the Internet to the external location.

It will be appreciated that the system and method described herein provides a mechanism by which data from an implanted cardiac stimulation device can be recorded over extended periods of time. Moreover, this data is not limited to simply a signal indicative of the function of the heart, but can also include other sensor signals received by the implanted cardiac stimulation device as well as marker data and state data of the cardiac stimulation device indicative of the manner in which the cardiac stimulation device is interpreting the signals that it is receiving.

It will be further appreciated that the method and system described herein further facilitates transfer of this data from the patient to an external location, such as a treating physician, as the data is stored either in a removable memory cartridge or is stored in such a manner that would permit subsequent downloading. Hence, the patient can continue normal daily activities wearing the monitor and transferring the data to the external location to thereby allow remote monitoring of the performance of the cardiac stimulation device. In this way, the flexibility and convenience of obtaining data over extended periods to allow for the evaluation of the performance of the implanted cardiac stimulation device is greatly enhanced. These and other objects and advantages of the present invention will become more apparent from the following description taken in conjunction with the accompanying drawings.

Preferably the method further comprises transmitting data relating to the function of the implantable cardiac stimulation device comprises transmitting data indicative of physiological signals obtained by the implantable cardiac stimulation device using physiological sensors, and preferably wherein transmitting indicative of physiological signals comprises transmitting data indicative of the metabolic need of the patient as obtained through a transthoracic impedance measurement. Preferably transmitting data indicative of physiological signals comprises transmitting data indicative of the metabolic need of the patient as obtained through a transthoracic impedance measurement.

Preferably, the method further comprises obtaining a signal using a sensor positioned external to the patient's body; and storing in the memory of the external recording system data indicative of signal obtained by the external sensor, and preferably obtaining a signal using the external sensor on the patient comprises measuring a physiological condition of the patient that corresponds to at least one of the signals being transmitted by the implantable cardiac stimulation device, and preferably providing the stored data to an external system comprises downloading the data to the external system comprises downloading the data to the external system via a communications link, and preferably providing the stored data to an external system comprises transmitting the data via a network to a remote location.

Preferably the method further comprises transmitting data relating to the function of the implantable cardiac stimulation device comprises transmitting data indicative of the markers determined by the implantable cardiac stimulation in response to sensed physiological conditions of the patient, and preferably transmitting data indicative of markers comprises transmitting data indicative of the implantable cardiac stimulation device operation, and preferably transmitting the data relating to the function of the implantable cardiac stimulation device comprises continuously transmitting the data over at least a 24-hour period, and preferably transmitting the data comprises transmitting the data via a telemetry circuit at a data transfer rate of approximately 8 bits per millisecond.

Embodiments of the invention will now be described, by way of example, with reference to the drawings, of which:
**Fig. 1** is a schematic illustration illustrating the components of the ambulatory recording device of the preferred embodiment used in conjunction with an implanted cardiac stimulation device;
**Fig. 2** is a block diagram of the ambulatory recording device of **Fig. 1;**
**Fig. 3** is an illustration of a typical implantable cardiac stimulation device that is used in conjunction with the ambulatory recording system of **Fig. 1;**
**Fig. 4** is a block diagram of the implantable cardiac stimulation device of **Fig. 3** and
**Fig. 5** is an exemplary flow chart illustrating the operation of the implantable cardiac stimulation device of **Figs. 3** and **4** as it communicates with the ambulatory recording device of **Fig. 1.**

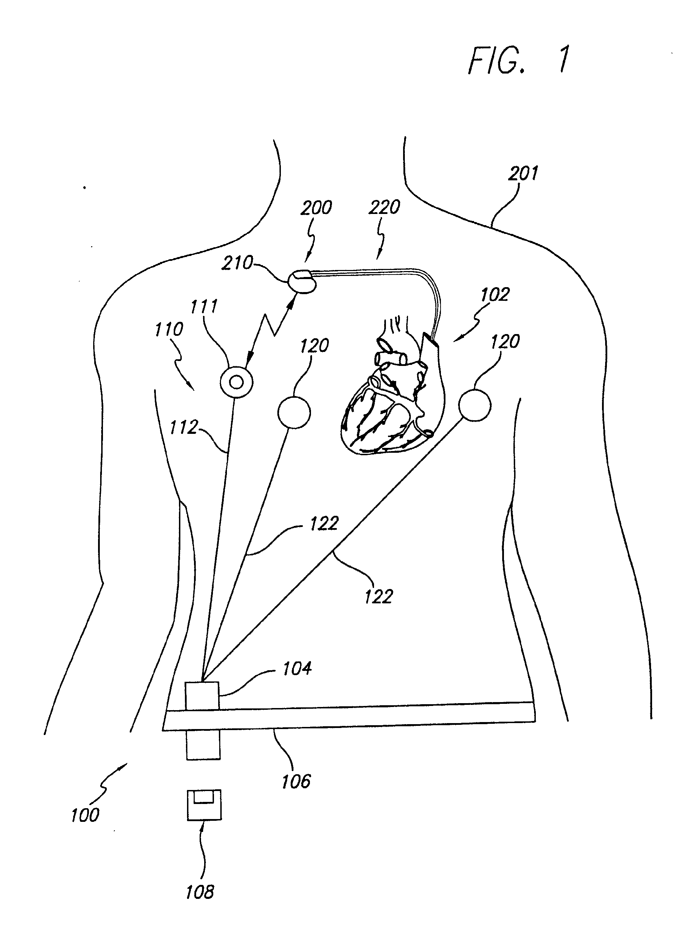

Reference will now be made to the drawings wherein like numerals refer to like parts throughout. **Fig. 1** generally illustrates the ambulatory recording device 100 of the illustrated embodiment. As shown in **Fig. 1,** the device 100 is adapted to communicate with a cardiac stimulation device 210 that is implanted within the body of a patient 201. The cardiac stimulation device 210 includes a housing 211 that contains the control circuitry and a plurality of leads 220 that extend into one or more of the chambers of the heart 102 in a known manner.

The ambulatory recording device 100 is adapted to communicate with the implanted cardiac stimulation device 210 so as to obtain signals from the implanted cardiac stimulation device 210 to thereby obtain a long-term record of the function of the patient's heart 102 and the operation of the implanted cardiac stimulation device 210. As is shown in **FIG. 1**, the ambulatory recording device 100 includes a housing 104 that is adapted, in this embodiment, to be attached to the belt 106 of the patient 201. The housing 104 includes the circuitry of the device and is relatively compact such that the user can wear the ambulatory recording device 100 during the course of their normal daily activities.

As is also illustrated in **FIG. 1**, the ambulatory recording device 100 can include a replaceable memory cartridge, such as a flash memory cartridge, an EE memory cartridge, a battery powered CMOS memory cartridge, a tape, a CD, a diskette or any other type of removable memory cartridge. The use of a removable memory cartridge 108 facilitates the transfer of large amounts of data from the ambulatory recording device 100 to a remote location, such as a treating physician's office. It will, however, be appreciated that the data transfer can occur in any of a number of fashions and that a removable memory cartridge is not a requirement for the operation of the ambulatory recording device 100.

As is also illustrated in **FIG. 1**, the ambulatory recording device 100 includes a communications wand 110 that is adapted to communicate with the control unit of the implanted cardiac stimulation device 210 in a known manner. The wand 110 preferably comprises a coil 111 which is attached to the ambulatory recording device housing 104 via a lead 112. The coil 111 is preferably secured in position on the outer skin of the patient 201 in a position where the coil 111 can communicate with the telemetry circuit of the implanted cardiac stimulation device in a manner that will be described in greater detail hereinbelow.

As is also illustrated in **FIG. 1**, the ambulatory recording device 100 can also optionally include one or more external sensors which, in this embodiment, are EKG patches 120 that are coupled to the housing 104 of the ambulatory recording device 100 via leads 122. The optional EKG patches 120 provide an EKG signal to the ambulatory recording device 100 which can then be stored and compared to the intracardiac electrogram (IEGM) signal that is being received by the implanted cardiac stimulation device 210 for comparison and verification purposes in the manner that will be described in greater detail below. While in this embodiment the external sensors comprise EKG electrodes, the ambulatory recording device 100 can be equipped with any of a number of different sensors, such as acceleration sensors, motion sensors and the like, in order to compare externally received sensor signals to signals that are being received by the microprocessor of the implanted device. In this way, the operation of the internal sensors implanted within the body of the patient can be compared to external signals to ensure that the implanted device sensors and/or algorithms are operating appropriately.

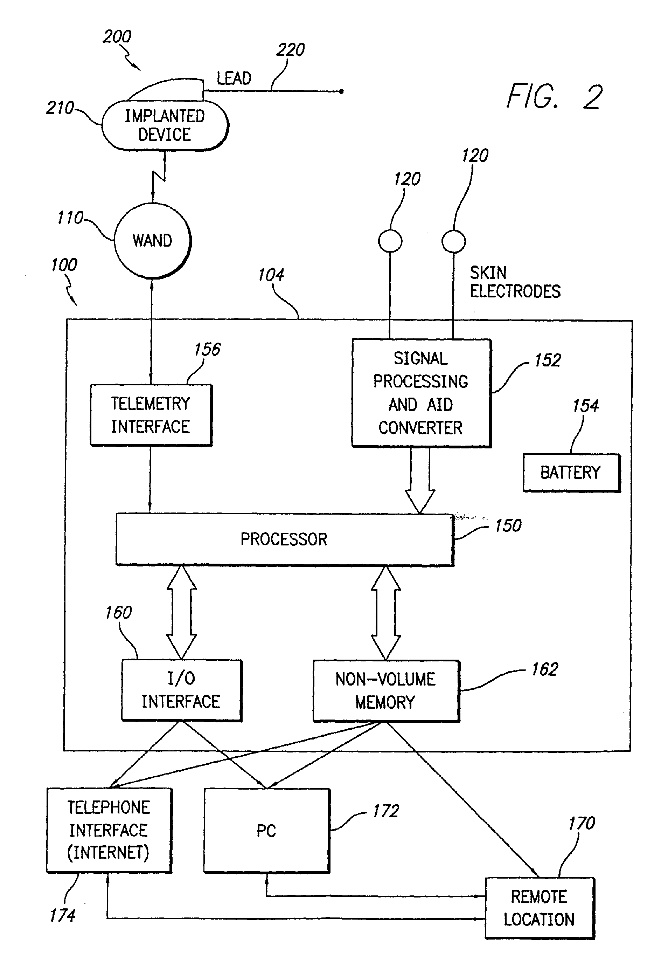

**FIG. 2** is a functional block diagram that illustrates the functional components of the ambulatory recording device 100. As is indicated in **FIG. 2**, the housing 104 includes a processor 150 that interfaces with the wand 110 via a known telemetry interface 156. In this way, the processor 150 can receive signals from the implanted device 210 via the wand 110 and the telemetry interface 156 and can store these signals in a non-volatile memory 162. As discussed above, the non-volatile memory 162 can comprise a removable memory cartridge or a non-removable memory device or can comprise any of a number of known non-volatile memories capable of storing data over an extended period of time. The processor also receives the EKG signal from the skin electrodes 120 via an Analog to Digital (A/D) converter 152 and these signals can also be stored in a non-volatile memory 162.

In one particular embodiment, the non-volatile memory 162 is comprised of a flash memory having a capacity of 134 MB which is capable of storing over 24 hours of continuous data being transmitted from the implanted cardiac stimulation device 210 at a transfer rate of 8 bits per millisecond. As is also illustrated in **FIG. 2**, the device 100 is powered by a battery 154 which is preferably sized to permit continuous operation of the device 100 over an extended period of time, e.g. greater than 24 hours. Since the non-volatile memory 162 and the battery 154 are preferably sized so as to permit continuous operation and storage of signals over an extended period of time and since the ambulatory recording device 100 is adapted to be carried by the user, substantially continuous data from the implanted cardiac stimulation device 210 can be obtained and recorded.

Similarly, if the optional EKG patches 120 are used, an EKG signal can also be stored in the non-volatile memory 162 for the same extended period of time. As will be discussed in greater detail below, the data that is being transmitted from the implanted cardiac stimulation device 210 can include sensor data that is being received by the implanted cardiac stimulation device 210, such as the intracardiac electrogram (IEGM) signal, signals from physiological sensors such as acceleration sensors, transthoracic impedance sensors, and the like. Moreover, internal data from the processor of the implanted cardiac stimulation device 210 can also be sent to the ambulatory recording device 100 thereby providing an indication as to the manner in which the implanted cardiac stimulation device 210 is evaluating and interpreting the data that it is receiving from the sensors and the manner in which the implantable stimulation device is providing therapy.

As is also illustrated in **FIG. 2**, the device 100 is preferably adapted to be able to transfer the stored data to a remote location 170. In one embodiment, a removable memory cartridge 108 is removed from the housing 104 of the ambulatory recording device 100 and is provided to the remote location 170 in any of a number of ways, including hand delivery, delivery by mail and the like. Alternatively, the information in the removable memory cartridge 108 may be loaded into a telephone interface 174, personal computer interface 172 or some other interface that is located in proximity to the patient 201 such that the data can then be transmitted electronically or telephonically to the remote location 170.

In another embodiment, the ambulatory recording device 100 is equipped with an I/O interface 160 that communicates with the processor 150 such that data stored in the non-volatile memory 162 can be downloaded via the I/O interface 160 to either a personal computer 172 or a telephone interface 174 in any of a number of known manners. Preferably, the I/O interface 160 is a high-speed interface, such as a parallel interface, such that data from the non-volatile memory 162 can be rapidly downloaded for subsequent transmission to the remote location 170. The I/O interface 160 can be configured in any of a number of ways such that it can transmit the data via known interfaces like a parallel, RS232 or USB interface to either the personal computer 172 or some other telephone interface device 174 for subsequent transmission to the remote location 170. The transmission to the remote location can either be via direct modem connections, Internet connection, or via any other communication medium.

Hence, the ambulatory recording device 100 enables the user to store large amounts of data indicative of not only the function of their heart, but also of the operation of the implanted cardiac stimulation device over an extended period of time. Moreover, this data can also be transferred to a treating physician or some other health care professional in a remote location 170 without requiring the patient to visit the remote location 170. This reduces the inconvenience of the patient associated with obtaining data as to the function of their heart and the performance of the implanted cardiac stimulation device over extended periods of time.

The ambulatory recording device 100 is adapted to be used with implanted cardiac stimulation devices such that data about the operation of the implanted cardiac stimulation device can be recorded over extended periods of time with reduced inconvenience to the patient.

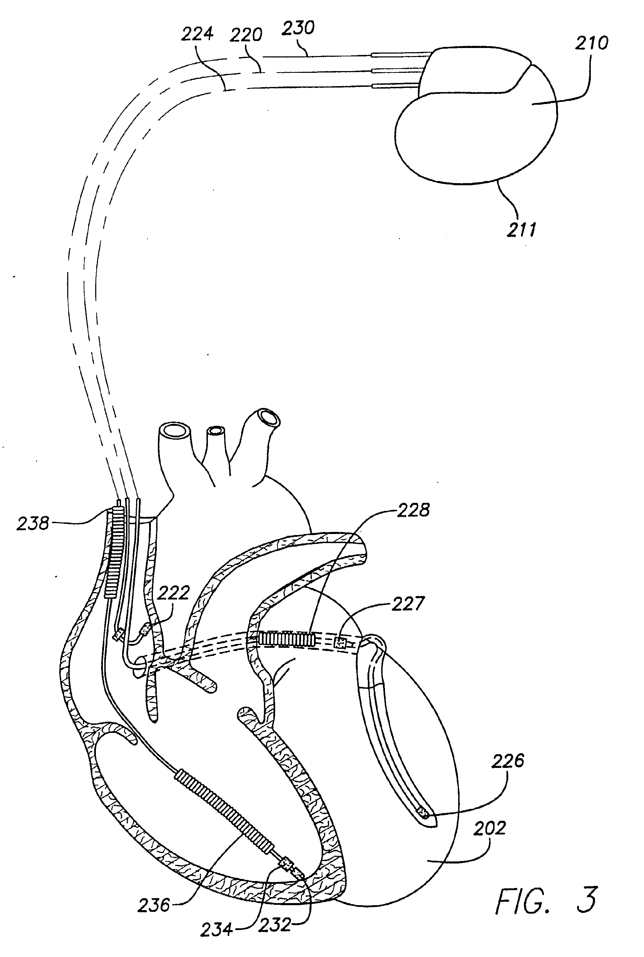

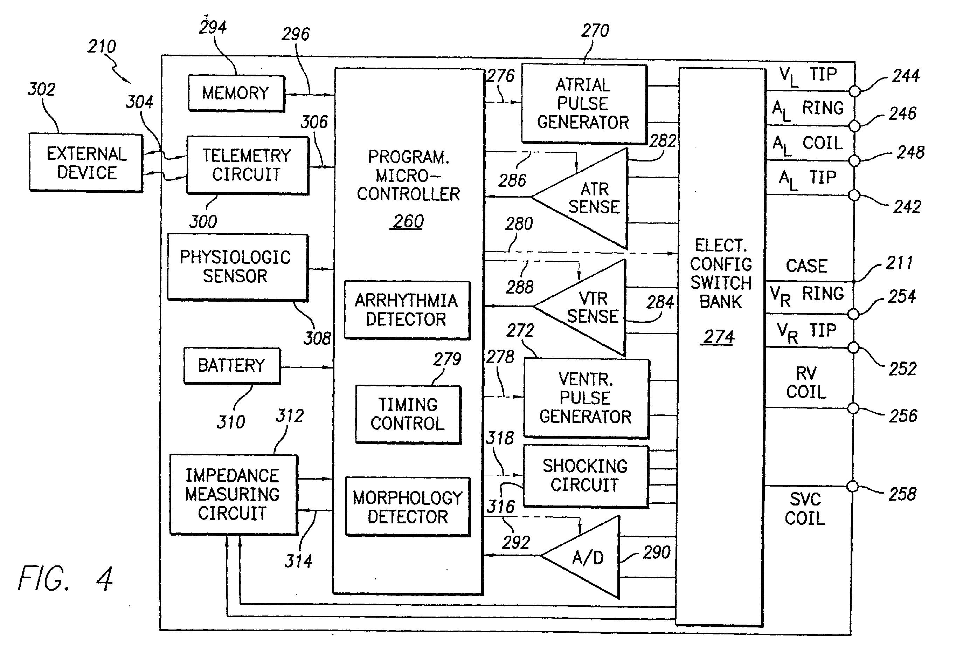

To fully comprehend the type and nature of the operating parameters and physiological signals that could be recorded by the present invention, it would be helpful to fully describe a state-of-the-art implantable cardiac stimulation device. To this end, **FIGS. 3 and 4** illustrate an exemplary implantable cardiac stimulation device of a type commonly implanted in patients today. The following description describes the basic operation parameters of these devices including the types of sensor signals received by these devices and the operating states of the implanted devices which are all signals that can be provided to the ambulatory recording device 100 of the illustrated embodiment in the manner described in greater detail hereinbelow.

As shown in **FIG. 3**, there is a stimulation device 210 in electrical communication with a patient's heart 102 by way of three leads, 220, 224 and 230, suitable for delivering multi-chamber stimulation and shock therapy. To sense atrial cardiac signals and to provide right atrial chamber stimulation therapy, the stimulation device 210 is coupled to an implantable right atrial lead 220 having at least an atrial tip electrode 222, which typically is implanted in the patient's right atrial appendage.

To sense left atrial and ventricular cardiac signals and to provide left chamber pacing therapy, the stimulation device 210 is coupled to a "coronary sinus" lead 224 designed for placement in the "coronary sinus region" via the coronary sinus os for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus.

Accordingly, an exemplary coronary sinus lead 224 is designed to receive atrial and ventricular cardiac signals and to deliver left ventricular pacing therapy using at least a left ventricular tip electrode 226, left atrial pacing therapy using at least a left atrial ring electrode 227, and shocking therapy using at least a left atrial coil electrode 228. For a complete description of a coronary sinus lead, see U.S. Patent Application No. 09/457,277, "A Self-Anchoring, Steerable Coronary Sinus Lead" (Pianca et al.), and U.S. Patent No. 5,466,254, "Coronary Sinus Lead with Atrial Sensing Capability" (Helland)

The stimulation device 210 is also shown in electrical communication with the patient's heart 102 by way of an implantable right ventricular lead 230 having, in this embodiment, a right ventricular tip electrode 232, a right ventricular ring electrode 234, a right ventricular (RV) coil electrode 236, and an SVC coil electrode 238. Typically, the right ventricular lead 230 is transvenously inserted into the heart 102 so as to place the right ventricular tip electrode 232 in the right ventricular apex so that the RV coil electrode will be positioned in the right ventricle and the SVC coil electrode 238 will be positioned in the superior vena cava. Accordingly, the right ventricular lead 230 is capable of receiving cardiac signals and delivering stimulation in the form of pacing and shock therapy to the right ventricle.

As illustrated in **FIG. 4**, a simplified block diagram is shown of the multi-chamber implantable stimulation device 210, which is capable of treating both fast and slow arrhythmias with stimulation therapy, including cardioversion, defibrillation, and pacing stimulation. While a particular multi-chamber device is shown, this is for illustration purposes only, and one of skill in the art could readily duplicate, eliminate or disable the appropriate circuitry in any desired combination to provide a device capable of treating the appropriate chamber(s) with cardioversion, defibrillation and pacing stimulation.

The housing 211 for the stimulation device 210, shown schematically in **FIG. 4**, is often referred to as the "can", "case" or "case electrode" and may be programmably selected to act as the return electrode for all "unipolar" modes. The housing 211 may further be used as a return electrode alone or in combination with one or more of the coil electrodes, 228, 236 and 238, for shocking purposes. The housing 211 further includes a connector (not shown) having a plurality of terminals, 242, 244, 246, 248, 252, 254, 256, and 258 (shown schematically and, for convenience, the names of the electrodes to which they are connected are shown next to the terminals). As such, to achieve right atrial sensing and pacing, the connector includes at least a right atrial tip terminal (A_{R} TIP) 242 adapted for connection to the atrial tip electrode 222.

To achieve left chamber sensing, pacing and shocking, the connector includes at least a left ventricular tip terminal (V_{L} TIP) 244, a left atrial ring terminal (A_{L} RING) 246, and a left atrial shocking terminal (A_{L} COIL) 248, which are adapted for connection to the left ventricular ring electrode 226, the left atrial tip electrode 227, and the left atrial coil electrode 228, respectively.

To support right chamber sensing, pacing and shocking, the connector further includes a right ventricular tip terminal (V_{R} TIP) 252, a right ventricular ring terminal (V_{R} RING) 254, a right ventricular shocking terminal (R_{V} COIL) 256, and an SVC shocking terminal (SVC COIL) 258, which are adapted for connection to the right ventricular tip electrode 232, right ventricular ring electrode 234, the RV coil electrode 236, and the SVC coil electrode 238, respectively.

At the core of the stimulation device 210 is a programmable microcontroller 260 which controls the various modes of stimulation therapy. As is well known in the art, the microcontroller 260 typically includes a microprocessor, or equivalent control circuitry, designed specifically for controlling the delivery of stimulation therapy and may further include RAM or ROM memory, logic and timing circuitry, state machine circuitry, and I/O circuitry. Typically, the microcontroller 260 includes the ability to process or monitor input signals (data) as controlled by a program code stored in a designated block of memory. The details of the design and operation of the microcontroller 260 are not critical to the present invention. Rather, any suitable microcontroller 260 may be used that carries out the functions described herein. The use of microprocessor-based control circuits for performing timing and data analysis functions are well known in the art.

As shown in **FIG. 4**, an atrial pulse generator 270 and a ventricular pulse generator 272 generate pacing stimulation pulses for delivery by the right atrial lead 220, the right ventricular lead 230, and/or the coronary sinus lead 224 via an electrode configuration switch 274. It is understood that in order to provide stimulation therapy in each of the four chambers of the heart, the atrial and ventricular pulse generators, 270 and 272, may include dedicated, independent pulse generators, multiplexed pulse generators, or shared pulse generators. The pulse generators, 270 and 272, are controlled by the microcontroller 260 via appropriate control signals, 276 and 278, respectively, to trigger or inhibit the stimulation pulses.

The microcontroller 260 further includes timing control circuitry 279 which is used to control the timing of such stimulation pulses (e.g., pacing rate, atrio-ventricular (AV) delay, atrial interconduction (A-A) delay, or ventricular interconduction (V-V) delay, etc.) as well as to keep track of the timing of refractory periods, PVARP intervals, noise detection windows, evoked response windows, alert intervals, marker channel timing, etc., which is well known in the art.

The switch 274 includes a plurality of switches for connecting the desired electrodes to the appropriate I/O circuits, thereby providing complete electrode programmability. Accordingly, the switch 274, in response to a control signal 280 from the microcontroller 260, determines the polarity of the stimulation pulses (e.g., unipolar, bipolar, combipolar, etc.) by selectively closing the appropriate combination of switches (not shown) as is known in the art.

Atrial sensing circuits 282 and ventricular sensing circuits 284 may also be selectively coupled to the right atrial lead 220, coronary sinus lead 224, and the right ventricular lead 230, through the switch 274 for detecting the presence of cardiac activity in each of the four chambers of the heart. Accordingly, the atrial (ATR. SENSE) and ventricular (VTR. SENSE) sensing circuits, 282 and 284, may include dedicated sense amplifiers, multiplexed amplifiers, or shared amplifiers. The switch 274 determines the "sensing polarity" of the cardiac signal by selectively closing the appropriate switches, as is also known in the art. In this way, the clinician may program the sensing polarity independent of the stimulation polarity.

Each sensing circuit, 282 and 284, preferably employs one or more low power, precision amplifiers with programmable gain and/or automatic gain control, bandpass filtering, and a threshold detection circuit, as known in the art, to selectively sense the cardiac signal of interest. The automatic gain control enables the device 210 to deal effectively with the difficult problem of sensing the low amplitude signal characteristics of atrial or ventricular fibrillation.

The outputs of the atrial and ventricular sensing circuits, 282 and 284, are connected to the microcontroller 260 which, in turn, are able to trigger or inhibit the atrial and ventricular pulse generators, 270 and 272, respectively, in a demand fashion in response to the absence or presence of cardiac activity in the appropriate chambers of the heart.

For arrhythmia detection, the device 210 utilizes the atrial and ventricular sensing circuits, 282 and 284, to sense cardiac signals to determine whether a rhythm is physiologic or pathologic. As used herein "sensing" is reserved for the noting of an electrical signal, and "detection" is the processing of these sensed signals and noting the presence of an arrhythmia. The timing intervals between sensed events (e.g., P-waves, R-waves, and depolarization signals associated with fibrillation which are sometimes referred to as "F-waves" or "Fib-waves") are then classified by the microcontroller 260 by comparing them to a predefined rate zone limit (i.e., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, anti-tachycardia pacing, cardioversion shocks or defibrillation shocks, collectively referred to as "tiered therapy").

Cardiac signals are also applied to the inputs of an analog-to-digital (A/D) data acquisition system 290. The data acquisition system 290 is configured to acquire intracardiac electrogram signals, convert the raw analog data into a digital signal, and store the digital signals for later processing and/or telemetric transmission to an external device, such as the ambulatory recording system 100. The data acquisition system 290 is coupled to the right atrial lead 220, the coronary sinus lead 224, and the right ventricular lead 230 through the switch 274 to sample cardiac signals across any pair of desired electrodes.

The microcontroller 260 is further coupled to a memory 294 by a suitable data/address bus 296, wherein the programmable operating parameters used by the microcontroller 260 are stored and modified, as required, in order to customize the operation of the stimulation device 210 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart 102 within each respective tier of therapy.

Advantageously, the operating parameters of the implantable device 210 may be non-invasively programmed into the memory 294 through a telemetry circuit 300 in telemetric communication with the external device, such as a programmer, transtelephonic transceiver, a diagnostic system analyzer or the ambulatory recording device 100. The telemetry circuit 300 is activated by the microcontroller by a control signal 306. The telemetry circuit 300 advantageously allows intracardiac electrograms and status information relating to the operation of the device 210 (as contained in the microcontroller 260 or memory 294) to be sent to the ambulatory recording device 100 through the wand 110 and telemetry interface 156 (**FIG. 2**). For examples of such devices, see U.S. Patent No. 4,809,697, entitled "Interactive Programming and Diagnostic System for Use with Implantable Pacemaker" (Causey, III et al.); U.S. Patent No. 4,944,299, entitled "High Speed Digital Telemetry System for Implantable Device" (Silvian); and U.S. Patent Application No. 09/223,422, filed 12/30/1998, entitled "Efficient Generation of Sensing Signals in an Implantable Medical Device such as a Pacemaker or ICD" (note: this relates to transfer of EGM data) (McClure et al.)

In the preferred embodiment, the stimulation device 210 further includes a physiologic sensor 308, commonly referred to as a "rate-responsive" sensor because it is typically used to adjust pacing stimulation rate according to the exercise state of the patient. However, the physiological sensor 308 may further be used to detect changes in cardiac output, changes in the physiological parameters (e.g., blood oxygen levels, stroke volume, contractility, etc.) or condition (e.g., CHF, etc.) of the heart, or diurnal changes in activity (e.g., detecting sleep, wake and exercise states). Accordingly, the microcontroller 260 responds by adjusting the various pacing parameters (such as rate, AV Delay, V-V Delay, etc.) at which the atrial and ventricular pulse generators, 270 and 272, generate stimulation pulses. While shown as being included within the stimulation device 210, it is to be understood that the physiologic sensor 308 may also be external to the stimulation device 210, yet still be implanted within or carried by the patient. A common type of rate responsive sensor is an activity sensor, such as an accelerometer or a piezoelectric crystal, which is mounted within the housing 211 of the stimulation device 210. Other types of physiologic sensors are also known, for example, sensors which sense the oxygen content of blood, respiration rate and/or minute ventilation, pH of blood, ventricular gradient, etc. However, any sensor may be used which is capable of sensing a physiological parameter, which corresponds to the exercise state of the patient. The type of sensor used is not critical to the present invention and is shown only for completeness.

The stimulation device additionally includes a battery 310, which provides operating power to all of the circuits shown in **FIG. 4.** As further shown in **FIG. 4**, the device 210 is shown as having an impedance measuring circuit 312 which is enabled by the microcontroller 260 via a control signal 314. The known uses for an impedance measuring circuit 312 include, but are not limited to, lead impedance surveillance during the acute and chronic phases for proper lead positioning or dislodgment, detecting operable electrodes and automatically switching to an operable pair if dislodgment occurs, measuring respiration or minute ventilation, measuring thoracic impedance for determining shock thresholds, detecting when the device has been implanted, measuring stroke volume, and detecting the opening of heart valves, etc. The impedance measuring circuit 312 is advantageously coupled to the switch 274 so that any desired electrode may be used. The impedance measuring circuit 312 is not critical to the present invention and is shown only for completeness.

In the case where the stimulation device 210 is intended to operate as an implantable cardioverter/defibrillator (ICD) device, it must detect the occurrence of an arrhythmia and automatically apply an appropriate electrical shock therapy to the heart aimed at terminating the detected arrhythmia. To this end, the microcontroller 260 further controls a shocking circuit 316 by way of a control signal 318. The shocking circuit 316 generates shocking pulses of low (up to 0.5 Joules), moderate (0.5 - 10 Joules), or high energy (11 to 40 Joules), as controlled by the microcontroller 260. Such shocking pulses are applied to the patient's heart 102 through at least two shocking electrodes, and as shown in this embodiment, selected from the left atrial coil electrode 228, the RV coil electrode 236, and/or the SVC coil electrode 238. As noted above, the housing 211 may act as an active electrode in combination with the RV electrode 236, or as part of a split electrical vector using the SVC coil electrode 238 or the left atrial coil electrode 228 (i.e., using the RV electrode as a common electrode).

Hence, as is illustrated in **FIGS. 3** and **4**, implantable cardiac stimulation devices can function in any of a number of different ways in providing differing therapy to the patient. The microcontroller 260 of the implantable cardiac stimulation device thus provides the therapy in response to the detection of certain conditions indicating that electrical stimulation is needed. The type and configuration of the electrical stimulation that is provided to the heart is, of course, dependent upon the configuration of the implanted device and the sensed condition that the electrical stimulation is designed to regulate. As a consequence, the microcontroller 260 receives signals indicative of the heart performance, i.e., an IEGM signal, and also other physiological signals that are indicative of the physiological condition of the patient. Based upon all of these signals, the microcontroller is programmed to provide an appropriate therapy to the heart in order to regulate heart function. The exact manner in which the microcontroller 260 decides to provide the therapy is dependent upon the algorithms that have been downloaded into the microcontroller and also upon the conditions that are sensed by the microcontroller. Hence, being able to record the signals received by the microcontroller 260, the state conditions of the microcontroller 260 or marker indications of the microcontroller 260 would thus allow a treating physician to determine whether the microcontroller 260 is programmed to provide the appropriate electrical stimulation therapy to the patient.

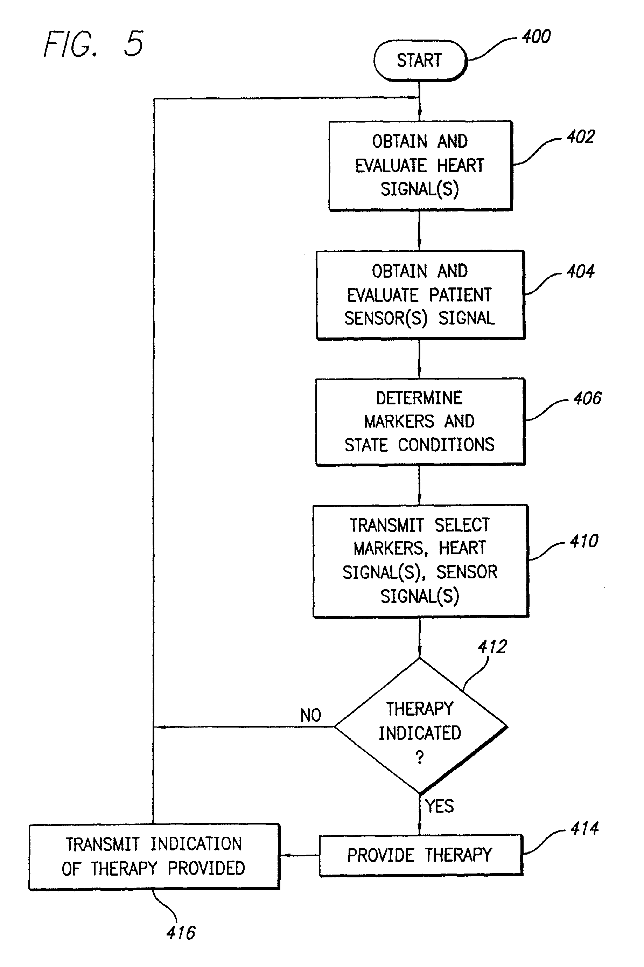

**FIG. 5** is a simplified flow chart illustrating the operation of the microcontroller 260 as it provides signals to the ambulatory recording device 100. It will be appreciated that the microcontroller 260 will initially have to be set so as to transmit particular patient sensor signals, marker signals, state information signals or heart signals to the ambulatory recording device 100 via the telemetry circuit 300. The programming to configure the programmable microcontroller 260 to transmit this information can be achieved via the telemetry circuit 300 through the use of a known external programmer. It will be further appreciated that any of a number of different signals, internal states of the microcontroller 260, markers and input signals to the microcontroller 260 can be programmed to be sent to the ambulatory recording device 100 without departing from the spirit of the present invention. Hence, the flow chart of **FIG. 5** is simply exemplary of the basic operational process of the microcontroller 260 when it has been programmed to either periodically or continuously download signals to the ambulatory recording device 100 over an extended period of time.

In basic operation, the programmable microcontroller 260, from a start state 400, initially obtains and evaluates the heart signal in state 402. As discussed above, the heart signal can comprise one or more signals indicative of the function of the heart that is being provided via the leads that are positioned within the heart. The exact configuration of the heart signal, of course, will vary depending upon the physiological condition of the patient and the type of devices implanted within the patient. The microcontroller 260 will also obtain and evaluate, in state 404, one or more patient sensor signals indicative of the physiological condition of the patient. These patient sensor signals can be generated by the physiological sensor 308 (**FIG. 4**) or the impedance measuring circuit 312. The patient sensor signals can, for example, comprise activity signals indicative of the activity level of the patient, the orientation of the patient, or the metabolic need of the patient (as measured by transthoracic impedance).

As is indicated in **FIG. 5**, the microcontroller 260 can then determine, in state 406, the various markers and state conditions based on the heart and patient sensor signals. As is understood in the art, the microcontroller 260 is programmed to make determinations based upon the heart signal and the various patient sensor signals. If the microcontroller 260 determines that a particular event has occurred, the microcontroller 260 then sets an appropriate marker indicative of the occurrence of a particular event. For example, if the implantable device 210 comprises a pacer, one marker may be an indication that an R-wave has occurred. The microcontroller 260 will then set this marker when it detects a heart signal component that meets a pre-selected threshold value and shape. Hence, markers are indications of whether the microcontroller 260 has determined, according to its programmed algorithm, whether a particular event has occurred and can include such things as the detection of R-waves, or P-waves, the occurrence of an appropriate refractory interval and various other known markers.

The microcontroller 260 is a state machine such that it is also determining particular values in accordance with its programmed algorithm. These values can include peak R-wave magnitude and the like. These values, along with determined markers and the like can also be sent to the ambulatory recording system 100 in the manner that will be described in greater detail hereinbelow.

Hence, the microcontroller 260 can be adapted to send signals, in state 410, selected by a treating or implanting physician that are indicative of the signals that the microcontroller 260 is receiving from the heart and also from other physiological sensors, as well as signals that are indicative of the determinations that the microcontroller 260 is making. This information can then be used to evaluate whether the microcontroller 260 is applying appropriate therapy to the patient.

The microcontroller 260 also evaluates the various markers and input signals to determine whether therapy is indicated in decision state 412. The determination as to whether therapy is indicated is dependent upon the programmed algorithm that has been initially downloaded into the microcontroller 260. If the microcontroller 260 concludes, in decision state 412, that therapy is indicated, the appropriate therapy is then provided in state 414. Basically, the microcontroller sends the appropriate therapy to the heart via the pulse generator circuits 270, 272 or the shocking circuit 316, the electrical configuration switch 274 and the various electrodes 242-258 as appropriately needed. Subsequently, the programmable microcontroller 260 can be programmed to transmit, in state 416, an indication of the therapy provided to the ambulatory recording device 100 via the telemetry circuit 300.

Hence, the ambulatory recording device 100 receives signals indicative of the inputs being provided to the microcontroller 260, the determinations that the microcontroller 260 is making as to whether therapy should be provided, and also the type of therapy that is being provided to the heart. This information is preferably being provided to the ambulatory recording device 100 on a continuous basis at a sampling rate of 8 bits per millisecond such that the ambulatory recording device receives a substantially continuous stream of data indicative of the function of the patient's heart, the operation of the implantable cardiac stimulation device 210 and the therapy that is being provided in response to the sensor input. In this embodiment, the microcontroller 260 is programmed to continuously communicate with the ambulatory recording device 100. The manner in which the microcontroller 260 communicates with the ambulatory recording device 100 is the same manner in which the microcontroller communicates with an external programmer of the prior art.

In this particular embodiment, the microcontroller 260 transmits and receives data from the ambulatory recording device 100 at a transfer rate of 8 bits per millisecond. This results in 64 bits every 8 milliseconds which approximately corresponds to one frame of heart signal data every 8 milliseconds. In this embodiment, there is a handshake protocol that occurs as each frame is transmitted to ensure that communication between the ambulatory recording device 100 and the microcontroller 260 does not result in undesired signals being transmitted therebetween. Hence, while the data transfer rate is 8 bits per millisecond, the actual data being transferred out to the ambulatory recording system 100 is somewhat less than 8 bits per millisecond given the handshake and other overhead requirements.

As discussed above, all of this information stored in the ambulatory recording device 100 can, in one embodiment, be provided to a remote location 170 to thereby allow the treating physician to determine whether the operational parameters of the implanted cardiac stimulation device 210 are appropriate for a particular patient. The treating physician can subsequently alter or change the prestored threshold values within the programmable microcontroller 260 or the algorithm by which therapy is being provided to improve the therapy that is being proved to the patient.

As discussed above, in one embodiment, the ambulatory recording device 100 also simultaneously records an external EKG signal via the skin electrodes 120. This signal can be compared to the IEGM signal and markers that is being received by the programmable microcontroller 260 of the implanted cardiac stimulation device 210 as a cross-reference. This cross-referencing between the EKG and the signal that is actually being received by the implanted cardiac stimulation device allows for further refinement of the evaluation of the incoming signal to the microcontroller 260 of the implanted cardiac stimulation device. It will be appreciated that any of a number of external sensors can be used in conjunction with the ambulatory recording system.

It will be appreciated from the foregoing that the ambulatory recording device of the illustrated embodiment is suitable for recording data as to the operation of an implanted medical device, such as a cardiac stimulation device, over an extended period of time. This data can thus be used to determine how the implanted cardiac stimulation device is responding to the heart function and, in particular, how it is providing therapeutic electrical stimulation to the heart. This data can also be transferred to a remote location, which allows for the collection of this data in a manner that is less intrusive to the patient.

Although the foregoing description of the invention has shown, described and pointed out the novel feature of the present invention, it will be understood that various omissions, substitutions and changes in the form of the detail of the apparatus as illustrated as well as the uses thereof may be made by those skilled in the art without departing from the spirit of the present invention. Consequently, the scope of the invention should not be limited to the foregoing discussion but should be defined by the appended claims.

## Claims

1. An ambulatory monitoring device for use with implantable cardiac stimulation devices comprising:
a housing adapted to be worn external to the patient's body;
a communications interface positioned in the housing that communicates with the implantable cardiac stimulation device and receives data therefrom;
a storage system positioned in the housing that stores the received data; and
a control system positioned in the housing and coupled to the communications interface and the storage system that controls the storage of the received data in the storage system.

2. The device of claim 1, wherein the control system stores data indicative of the function of the patient's heart, preferably an IEGM signal or marker data indicative of the function of the implantable cardiac stimulation device as it provides therapy to the patient's heart.

3. The device of claim 1, wherein the storage system comprises one or more memory modules capable of storing data for an extended period of time provided by the implantable cardiac stimulation device, preferably one or more flash memory modules that can be removed from the housing and physically provided to an external system.

4. The device of claim 1, further comprising an interface coupled to the control system that allows transfer of the stored data to an external system, preferably wherein the interface comprises a communications link that provides the stored data in a manner that permits transfer of the stored data to the external system via an electronic network, and preferably wherein the communications link that provides the stored data in a manner that permits transfer of the stored data to the external system via a telephony modem connection.

5. The device of claim 1, further comprising an external sensor coupled to the control system that measures an external signal wherein data indicative of the external signal is stored in a storage system, and wherein the communication interface further receives physiological data from the implantable cardiac stimulation device and wherein the control system stores this data in the storage system such that the data indicative of the external signal can be utilized in conjunction with the physiological data being received by the communications interface from the implantable cardiac stimulation device, and preferably wherein the external sensor comprises an external electrogram monitor that obtains an external electrogram signal indicative of the function of the patient's heart and the control system stores data indicative of the external electrogram signal in the storage system and wherein the ambulatory recording device is further recording data representative of an internal electrogram signal received by the implantable cardiac stimulation device to thereby permit use of data representative of the external electrogram signal in conjunction with the data representative of the internal electrogram signal.

6. An ambulatory monitoring device for use with implantable cardiac stimulation devices comprising:
a housing adapted to be worn external to the patient's body;
means for communicating with the implantable cardiac stimulation device so as to receive data therefrom indicative of the performance of the implantable cardiac stimulation device wherein the means for communicating with the implantable cardiac stimulation device is positioned within the housing;
means for storing the received data for an extended period of time wherein the means for storing the received data is positioned within the housing; and
means for providing the stored data to an external system wherein the means for providing the stored data to an external system is positioned within the housing.

7. The device of claim 6, wherein the means for communicating with the implantable cardiac stimulation device comprises a telemetry link that links with the telemetry circuit of the implantable cardiac stimulation device.

8. The device of claim 6, wherein the means for storing the received data comprises a memory and a processor wherein the processor controls the storage of the received data in the memory over an extended period.

9. The device of claim 6, wherein the stored data in the memory comprises data indicative of the physiological conditions sensed by the implantable cardiac stimulation device.

10. The device of claim 6, wherein the stored data comprises marker data indicative of the determinations made by the implantable cardiac stimulation device as to whether to apply electrical stimulation to the heart of the patient.

11. The device of claim 6 wherein the means for providing the stored data to an external system comprises memory cartridges that are removable from the housing so as to permit the memory cartridges to be physically provided to an external system.

12. The device of claim 6, wherein the means for providing the stored data to an external system comprises a communications interface that transfers the stored data to the external system, preferably wherein the output communications interface comprises a communications link that provides the stored data to permit the data to be transferred to the external system via a telephony modem connection, or wherein the output communications interface comprises a communications link that provides the stored data to the external system via an internet connection.

13. The device of claim 6, further comprising a means for obtaining an external physiological signal indicative of the physiological condition of the patient, and preferably wherein the means for storing the received further data stores data indicative of the external physiological signal, for example physiological data obtained by the implantable cardiac stimulating device corresponding to the external physiological signal to permit subsequent use of the physiological data obtained by the implantable cardiac stimulating device in conjunction with the data indicative of the external physiological signal, and preferably wherin the means for obtaining an external physiological signal comprises an external electrogram monitor and wherein the implantable cardiac stimulation device receives an internal electrogram signal, and preferably, wherein the stored data in the means for storing the received data further comprises marker data from the implantable cardiac stimulation device.

14. A method of obtaining information from an implantable cardiac stimulating device in a patient for an extended period of time while the patient is engaged in normal daily activities, the method comprising:
mounting an external recording system on the patient; transmitting data relating to the function of the implantable cardiac stimulation device to the external recording system over the extended period of time; and
storing the data in a memory of the external recording system.
